# EUROPÄISCHE PATENTSCHRIFT

(11) **EP 2 379 866 B1**
(45) Veröffentlichungstag und Bekanntmachung des Hinweises auf die Patenterteilung: **27.09.2017**
(21) Anmeldenummer: 09799607.8
(22) Anmeldetag: 15.12.2009
(51) Int. Cl.: F02D 41/00, F02D 41/14, F02D 41/24

(54) **VERFAHREN UND VORRICHTUNG ZUM KALIBRIEREN EINES SENSORS**
METHOD AND DEVICE FOR CALIBRATING A SENSOR
PROCÉDÉ ET DISPOSITIF DE CALIBRAGE D'UN CAPTEUR

(30) Priorität: 16.12.2008 DE 102008062550
(43) Veröffentlichungstag der Anmeldung: 26.10.2011
(73) Patentinhaber: Continental Automotive GmbH, 30165 Hannover (DE)
(72) Erfinder: BIERL, Rudolf, 93055 Regensburg (DE); HEINRICH, Stephan, 84076 Pfeffenhausen (DE); WILDGEN, Andreas, 93152 Nittendorf (DE)
(86) Internationale Anmeldenummer: PCT/EP2009/067200
(87) Internationale Veröffentlichungsnummer: WO 2010/076196

(56) Entgegenhaltungen:
- EP-A2- 1 074 728
- DE-A1- 4 025 544
- JP-A- 2003 148 196
- JP-A- 2004 044 600
- US-A1- 2003 106 544

## Beschreibung

Die Erfindung betrifft ein Verfahren und eine Vorrichtung zum Kalibrieren eines Sensors. Beim Betrieb einer Brennkraftmaschine entfällt ein Großteil von emittierten Schadstoffen auf verbrennungsbedingt entstehendes Abgas. Darüber hinaus gibt es andere Quellen, die für Schadstoffemissionen verantwortlich sind. Zu diesen Quellen können beispielsweise Ausdünstungen eines Kraftstofftanks der Brennkraftmaschine gezählt werden.

Kraftstoffe wie beispielsweise Superbenzin, die in dem Kraftstofftank gelagert sein können, weisen eine Reihe von leicht-flüchtigen Kohlenwasserstoffen auf. Hierzu zählen beispielsweise Methan, Butan und Propan. Um den Kraftstofftank bei einer Änderung eines Volumens des Kraftstoffs vor mechanischen Schäden zu schützen und einen Druckausgleich zwischen dem Kraftstofftank und der Umluft zu ermöglichen, kann der Kraftstofftank über eine Leitung mit der Umluft gekoppelt sein. Die leicht-flüchtigen Kohlenwasserstoffe können sich insbesondere bei erhöhten Außentemperaturen, beispielsweise durch Sonneneinstrahlung, oder auch durch ein Schütteln des Kraftstofftanks während einer Fahrt aus dem Kraftstoff lösen und als gasförmige Bestandteile den Kraftstofftank über die Leitung verlassen.

Aus der JP 2003-148196 A ist eine Brennkraftmaschine mit einem Tankentlüftungssystem bekannt mit einem Dreiwegeventil, das in einem ersten Zustand einen Kohlenwasserstoffsensor mit einem Filter koppelt, wobei in diesem Zustand Spülgas von dem Filter in den Kohlenwasserstoffsensor hineinströmt. In einem zweiten Zustand des Dreiwegeventils koppelt dieses eine Neuluftroute mit dem Kohlenwasserstoffsensor und zwar derart, dass die Luft aus der Neuluftroute in den Kohlenwasserstoffsensor strömt. Zum Kalibrieren des Kohlenwasserstoffsensors wird das Dreiwegeventil in seinem zweiten Zustand gesteuert. Wenn festgestellt wurde, dass eine Entlüftung einer Gasprüfkammer des Kohlenwasserstoffsensors anschließend abgeschlossen wurde, wird abhängig von einem dann erfassten Messwert des Kohlenwasserstoffsensors und einem Referenzwert ein Korrekturwert ermittelt.

Die Aufgabe, die der Erfindung zugrunde liegt, ist es, ein Verfahren und eine Vorrichtung zu schaffen mit dem beziehungsweise der ein Sensor eines Tankentlüftungssystems, der ausgebildet ist zum Erfassen einer Kohlenwasserstoffkonzentration eines Fluids einfach und zuverlässig kalibriert werden kann.

Die Aufgabe wird gelöst durch die Merkmale der unabhängigen Ansprüche. Vorteilhafte Ausgestaltungen der Erfindung sind in den Unteransprüchen angegeben.

Gemäß einem Aspekt zeichnet sich die Erfindung aus durch ein Verfahren und eine korrespondierende Vorrichtung zum Kalibrieren eines Sensors eines Tankentlüftungssystems. Das Tankentlüftungssystem ist in einer Brennkraftmaschine angeordnet, die einen Ansaugtrakt aufweist. Das Tankentlüftungssystem umfasst einen Kohlenwasserstoffspeicher sowie ein Ventil, wobei das Ventil und der Sensor in einer Fluidleitung angeordnet sind. Die Fluidleitung koppelt den Kohlenwasserstoffspeicher mit dem Ansaugtrakt. Der Sensor ist ausgebildet zum Erfassen einer Kohlenwasserstoffkonzentration eines Fluids in der Fluidleitung. Wenn das Ventil geöffnet ist, wird mittels des Sensors ein erfasster Wert der Kohlenwasserstoffkonzentration erfasst. Bei dem geöffneten Ventil wird ein durch die Fluidleitung in den Ansaugtrakt strömender Massenstrom des Fluids erfasst oder ermittelt. Der Massenstrom kann die Kohlenwasserstoffkonzentration eines Luft-/KraftstoffGemisches, das der Brennkraftmaschine zur Verbrennung zugemessen wird, beeinflussen. Bei dem geöffneten Ventil wird von dem Luft-/Kraftstoff-Gemisch ein Luft-/Kraftstoff-Verhältnis ermittelt. Insbesondere werden der erfasste Wert der Kohlenwasserstoffkonzentration, der Massenstrom und das Luft-/ Kraftstoff-Verhältnis derart zeitnah zueinander erfasst beziehungsweise ermittelt, dass die tatsächliche Kohlenwasserstoffkonzentration während des Erfassens beziehungsweise Ermittelns weitgehend unverändert ist. Abhängig von dem Massenstrom des Fluids, dem Luft-/Kraftstoff-Verhältnis, einem Massenstrom eingespritzten Kraftstoffs (mK) und einem Massenstrom zugemessener Luft (mL) wird ein geschätzter Wert der Kohlenwasserstoffkonzentration in der Fluidleitung ermittelt. Der Sensor wird kalibriert abhängig von dem erfassten Wert der Kohlenwasserstoffkonzentration und dem geschätzten Wert der Kohlenwasserstoffkonzentration. Dies ermöglicht eine hohe Langzeitstabilität von Messsignalen des Sensors dadurch, dass der Sensor zuverlässig kalibriert werden kann.
Gemäß einer vorteilhaften Ausgestaltung wird das Luft-/Kraftstoff-Verhältnis abhängig von einem Messsignal einer Abgassonde in einem Abgastrakt der Brennkraftmaschine ermittelt. Bei der Abgassonde kann es sich beispielsweise um eine Lambdasonde handeln. Dies kann eine Installation von zusätzlichen Messvorrichtungen obsolet machen.

In einer weiteren vorteilhaften Ausgestaltung wird ermittelt, ob das Ventil zumindest seit einer vorgegebenen Zeitdauer geöffnet ist und falls das Ventil zumindest seit der vorgegebenen Zeitdauer geöffnet ist, wird ein weiterer erfasster Wert der Kohlenwasserstoffkonzentration mittels des Sensors erfasst. Der Sensor wird kalibriert abhängig von dem weiteren erfassten Wert der Kohlenwasserstoffkonzentration und einem vorgegebenen Referenzwert. Dies ermöglicht eine zuverlässige Kalibrierung des Sensors unabhängig von einer anderen Messvorrichtung.

Gemäß einem nicht erfindungsgemäßen Aspekt ist ein Verfahren und eine korrespondierende Vorrichtung zum Kalibrieren eines Sensors eines Tankentlüftungssystems offenbart. Das Tankentlüftungssystem ist in einer Brennkraftmaschine angeordnet, die einen Ansaugtrakt aufweist. Das Tankentlüftungssystem umfasst einen Kohlenwasserstoffspeicher sowie ein Ventil, wobei das Ventil und der Sensor in einer Fluidleitung angeordnet sind. Die Fluidleitung koppelt den Kohlenwasserstoffspeicher mit dem Ansaugtrakt. Der Sensor ist ausgebildet ist zum Erfassen einer Kohlenwasserstoffkonzentration eines Fluids in der Fluidleitung. Es wird ermittelt, ob das Ventil zumindest seit einer vorgegebenen Zeitdauer geöffnet ist. Falls das Ventil zumindest seit der vorgegebenen Zeitdauer geöffnet ist wird ein weiterer erfasster Wert der Kohlenwasserstoffkonzentration mittels des Sensors erfasst. Der Sensor wird kalibriert, abhängig von dem weiteren erfassten Wert der Kohlenwasserstoffkonzentration und einem vorgegebenen Referenzwert.

In einer vorteilhaften Ausgestaltung des erfindungsgemäßen Aspekts ist der vorgegebene Referenzwert charakteristisch für eine minimale Kohlenwasserstoffkonzentration in der Fluidleitung. Dies ermöglicht eine einfache und zuverlässige Kalibrierung des Sensors auf einen Minimalwert.

Ausführungsbeispiele der Erfindung sind im Folgenden anhand der schematischen Zeichnungen näher erläutert. Es zeigen:
- Figur 1: eine Brennkraftmaschine mit einem Tankentlüftungssystem,
- Figur 2: eine Steuervorrichtung,
- Figur 3: ein Ablaufdiagramm,
- Figur 4: eine Gleichung.

Elemente gleicher Konstruktion oder Funktion sind figurenübergreifend mit den gleichen Bezugszeichen gekennzeichnet.

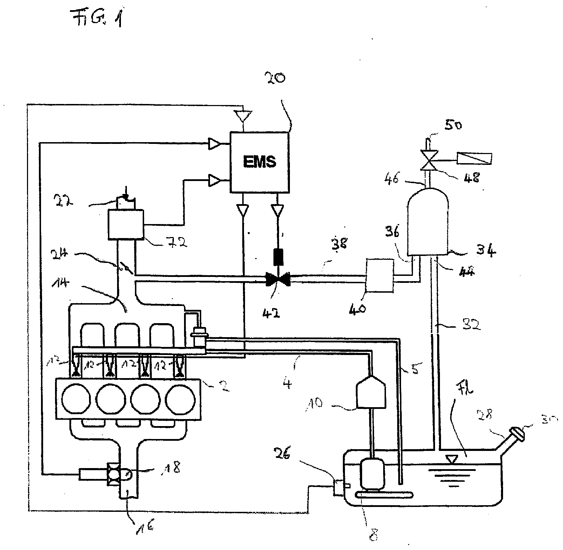

Figur 1 zeigt eine Brennkraftmaschine mit einem Motorblock 2, der durch eine Kraftstoffzuleitung 4 und eine Rückflussleitung 5 hydraulisch mit einem Tank 6 gekoppelt ist. Bei dem Tank 6 kann es sich beispielsweise um einen Kraftstofftank handeln, der mit Kraftstoff gefüllt sein kann. In dem Tank 6 ist eine Kraftstofffördereinheit 8 angeordnet, die Kraftstoff über die Kraftstoffzuleitung 4 und einen in der Kraftstoffzuleitung 4 angeordneten Filter 10 zu Einspritzventilen 12 leitet, die an dem Motorblock 2 angeordnet sind. Von den Einspritzventilen 12 wird der zugeführte Kraftstoff in den Motorblock 2 zugemessen, wo er zusammen mit Luft, die mittels eines Ansaugtrakts 14 in einem vorgegebenen Verhältnis zu dem Kraftstoff zugemessen wird, zur Verbrennung kommt. Entstehende Abgase des Verbrennungsprozesses werden durch einen Abgastrakt 16 von dem Motorblock 2 weggeführt. In dem Abgastrakt 16 ist eine Lambdasonde 18 angeordnet, die dazu ausgebildet ist ein Messsignal zu erzeugen, das repräsentativ ist für ein Luft-/Kraftstoff-Verhältnis LKV vor der Verbrennung. Die Lambdasonde 18 ist elektrisch gekoppelt mit einer Steuervorrichtung 20 und so bevorzugt Teil einer Lambdaregelung. Die Lambdasonde 18 kann auch als Abgassonde bezeichnet werden. Die Steuervorrichtung 20 kann als Vorrichtung zum Betreiben einer Brennkraftmaschine bezeichnet werden und beispielsweise als Motorsteuergerät ausgebildet sein. Dem Ansaugtakt 14 wird die Luft über einen Lufteinlass 22 zugeführt, in dem eine Drosselklappe 24 angeordnet ist.

In dem Tank 6 ist ferner ein Kraftstoffsensor 26 angeordnet. Bei dem Kraftstoffsensor 26 kann es sich beispielsweise um einen sogenannten Flex-Fuel-Sensor handeln, mittels dem eine Zusammensetzung des Kraftstoffs ermittelt werden kann. Aus dem Kraftstoff können, insbesondere bei erhöhten Umgebungstemperaturen, leicht-flüchtige Kohlenwasserstoffe abdampfen. Hierdurch entsteht in dem Tank 6 ein mit Kohlenwasserstoffen angereichertes Luft-/Kraftstoff-Gemisch, welches im Folgenden als Fluid Fl bezeichnet wird.

Der Tank 6 weist einen Einfüllstutzen 28 auf, an dessen Ende er mit einem Tankverschluss 30 hermetisch nach außen abgeschlossen ist. Bei einem Betrieb der Brennkraftmaschine kann sich das Volumen des in dem Tank 6 gespeicherten Kraftstoffs verringern, beispielsweise durch Entnahme von Kraftstoff mittels der Kraftstofffördereinheit 8. Es ist beispielsweise auch eine Vergrößerung des Kraftstoffvolumens in dem Tank 6 möglich, beispielsweise durch temperaturbedingte Ausdehnungen des Kraftstoffs bei hohen Außentemperaturen und längeren Stillstandszeiten der Brennkraftmaschine. Um einer Beschädigung des Tanks 6 wirksam entgegenzuwirken, ist der Tank 6 für einen Druckausgleich zwischen dem Tank 6 und der Umluft bei einer Volumenänderung des Tankinhaltes mit einer Lüftungsleitung 32 gekoppelt. Kommt es zu Volumenänderungen des Tankinhaltes, dann kann das mit Kohlenwasserstoffen angereicherte Fluid Fl in die Lüftungsleitung 32 gelangen. Es ist beispielsweise auch möglich, dass Kraftstoff in die Lüftungsleitung 32 gelangt, beispielsweise aufgrund von Erschütterungen eines Fahrzeugs, in dem die Brennkraftmaschine mit dem Tank 6 angeordnet ist. In diesem Fall ist der Anteil an Kohlenwasserstoff in dem Fluid Fl besonders groß.

Zur Filterung der in dem Fluid Fl enthaltenen Kohlenwasserstoffe weist die Brennkraftmaschine einen Kohlenwasserstoffspeicher 34 auf. Der Kohlenwasserstoffspeicher 34 kann beispielsweise als Aktivkohlefilter ausgebildet sein und ist dazu ausgebildet Kohlenwasserstoffe zu absorbieren und zu speichern. Der Kohlenwasserstoffspeicher 34 weist drei Anschlüsse auf. Ein erster Anschluss 36 ist mit einer Spülleitung 38 gekoppelt, die auch allgemein als Fluidleitung bezeichnet werden kann und über einen Sensor 40 und ein Ventil 42 mit dem Ansaugtrakt 14 gekoppelt ist. Über einen zweiten Anschluss 44 und die Lüftungsleitung 32 ist der Kohlenwasserstoffspeicher 34 mit dem Tank 6 gekoppelt. Ein dritter Anschluss 46 koppelt den Kohlenwasserstoffspeicher 34 über ein Spülventil 48 mit einem Spüllufteinlass 50.

In einer vorteilhaften Ausgestaltung ist das Ventil 42 als analoges Ventil 42 ausgebildet. Dies ermöglicht eine stufenlose Vorgabe von Offenpositionen des Ventils 42. Insbesondere können beispielsweise im Gegensatz zu einem digital ausgebildeten Ventil Pulsationen in der Spülleitung 38 wirksam vermieden werden.

Das Absorptionsvermögen des Kohlenwasserstoffspeichers 34 bezüglich der Kohlenwasserstoffe ist begrenzt. Gelangt der Kohlenwasserstoffspeicher 34 in eine Sättigung, so wird er gespült. Hierzu werden das Spülventil 48 und das Ventil 42 geöffnet, so dass über den Spüllufteinlass 50 und das Spülventil 48 Umluft in den Kohlenwasserstoffspeicher 34 gelangt, die die in dem Kohlenwasserstoffspeicher 34 gespeicherten Kohlenwasserstoffe aufnimmt und über den Sensor 40 und die Spülleitung 38 dem Ansaugtrakt 14 der Brennkraftmaschine zumisst. Das Spülventil 48 und das Ventil 42 können dazu von der Steuervorrichtung 20 angesteuert werden. Die Anordnung des Kohlenwasserstoffspeichers 34 mit der Fluidleitung 38, dem Sensor 40 und dem Ventil 42 kann auch als Tankentlüftungssystem bezeichnet werden.

Zur Reduktion von Schadstoffen der Brennkraftmaschine und auch für eine Leistungsoptimierung wird das Luft-/Kraftstoff-Verhältnis LKV des Luft-/Kraftstoff-Gemisches vorgegeben. Das mit Kohlenwasserstoff aus dem Kohlenwasserstoffspeicher 34 angereicherte Fluid Fl gelangt über das Ventil 42 in den Ansaugtrakt 14, wo es das Luft-/Kraftstoff-Gemisch in seiner Zusammensetzung bezüglich des Kohlenwasserstoffanteils beeinflussen kann. Um das Luft-/Kraftstoff-Verhältnis LKV präzise vorgeben zu können, wird eine Kohlenwasserstoffkonzentration cHC des mit Kohlenwasserstoffen angereicherten Fluids Fl mittels des Sensors 40 erfasst. Für eine bekannte Kohlenwasserstoffkonzentration cHC des Fluids Fl können dann die Zufuhr der Luft aus dem Lufteinlass 22 und des Kraftstoffs entsprechend angepasst werden. Es kann beispielsweise auch ein Massenstrom mFl des mit Kohlenwasserstoffen angereicherten Fluids Fl mittels des Ventils 42 angepasst werden, beispielsweise mittels der Steuervorrichtung 20. Um auch über lange Zeiträume zuverlässige Messergebnisse des Sensors 40 erwarten zu können, kann der Sensor 40 kalibriert werden, beispielsweise in regelmäßigen Abständen. Bevorzugt wird der Sensor 40 in Abhängigkeit von mindestens zwei Messpunkten kalibriert. Dies ermöglicht eine Korrektur des Messsignals hinsichtlich einer Nullpunktsverschiebung und einer Verschiebung einer Steigung des Messsignals.

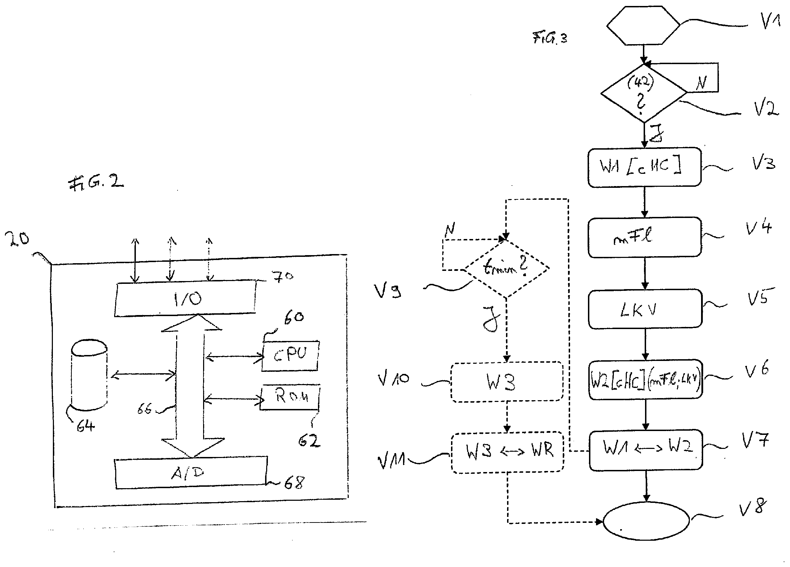

Figur 2 zeigt die Steuervorrichtung 20. Die Steuervorrichtung 20 umfasst einen Prozessor 60, einen Programmspeicher 62 sowie einen Datenspeicher 64. Der Prozessor 60, der Programmspeicher 62 sowie der Datenspeicher 64 sind gekoppelt miteinander über einen Systembus 66.

Die Steuervorrichtung 20 ist ausgebildet zur Ausführung eines Programms, das beispielsweise in dem Programmspeicher 62 gespeichert ist. Mittels des Programms kann beispielsweise der Sensor 40 kalibriert werden. Die Steuervorrichtung 20 kann daher ebenfalls als Vorrichtung zum Kalibrieren des Sensors 40 bezeichnet werden. Der Datenspeicher 64 ist ausgebildet zur Speicherung von Daten, beispielsweise der Messsignale.

Der Systembus 66 ist gekoppelt mit einem Analog-Digital-Wandler 68. Über den Analog-Digital-Wandler 68 ist die Steuervorrichtung 20 mit dem Kraftstoffsensor 26 und dem Sensor 40 zum Erfassen der Kohlenwasserstoffkonzentration cHC gekoppelt. Die empfangenen Messsignale können beispielsweise in dem Datenspeicher 64 gespeichert und von dem Prozessor 60 verarbeitet werden. Ferner weist die Steuervorrichtung 20 eine Schnittstelle 70 auf. Über die Schnittstelle 70 ist die Steuervorrichtung 20 zur Ansteuerung elektrisch gekoppelt mit den Einspritzventilen 12, dem Ventil 42 und dem Spülventil 50. Ferner ist die Steuervorrichtung 20 über die Schnittstelle 70 elektrisch mit der Lambdasonde 18 gekoppelt.

Figur 3 zeigt ein Ablaufdiagramm des beispielsweise von der Steuervorrichtung 20 ausgeführten Programms. Das Programm umfasst elf Schritte.

Das Programm beginnt in einem ersten Schritt V1. In dem ersten Schritt V1 können Parameter Initialisiert werden.

In einem zweiten Schritt V2 wird ermittelt, ob das Ventil 42 geöffnet ist. Wenn das Ventil geöffnet ist, fährt das Programm in einem dritten Schritt V3 fort. Wenn das Ventil 42 geschlossen ist, fährt das Programm in dem zweiten Schritt V2 fort.

In dem dritten Schritt V3 wird ein erfasster Wert W1 der Kohlenwasserstoffkonzentration cHC mittels des Sensors 40 erfasst.

In dem vierten Schritt V4 wird der Massenstrom mFl des durch die Fluidleitung 38 in den Ansaugtrakt 14 strömenden Fluids Fl erfasst oder ermittelt. Beispielsweise kann der Massenstrom mFl mit einem Fluidmassensensor erfasst werden. Es ist jedoch beispielsweise auch möglich, dass der Sensor 40 dazu ausgebildet ist, neben der Kohlenwasserstoffkonzentration cHC auch den Massenstrom mFl des Fluids Fl zu erfassen. Für den Fall, dass kein geeigneter Sensor in der Fluidleitung 38 angeordnet ist, kann der Massenstrom mFl beispielsweise abhängig von einem Druckunterschied zwischen der Fluidleitung 38 und dem Ansaugtrakt 14 der Brennkraftmaschine unter weiterer Berücksichtigung eines Durchmessers der Fluidleitung 38 ermittelt werden. Beispielsweise kann zum Ermitteln des Massenstroms mFl des Fluids Fl auch eine Temperatur berücksichtigt werden. Durch den Druckunterschied kann das mit Kohlenwasserstoff angereicherte Fluid Fl von der Fluidleitung 38 in den Ansaugtrakt 14 strömen.

In einem fünften Schritt V5 wird ein Luft-/Kraftstoff-Verhältnis LKV eines Luft-/Kraftstoff-Gemisches ermittelt. Das Luft-/Kraftstoff-Gemisch ist ausgebildet zur Verbrennung in der Brennkraftmaschine und setzt sich zusammen aus der Luft, die durch den Lufteinlass 22 eingesaugt wurde, aus dem durch die Einspritzventile 12 zugemessenen Kraftstoff sowie aus dem mit Kohlenstoff angereicherten Fluid Fl. Das Luft-/Kraftstoff-Verhältnis LKV kann beispielsweise nach der Verbrennung des Luft-/Kraftstoff-Gemisches in dem Abgastrakt 16 ermittelt werden, beispielsweise mittels der Lambdasonde 18.

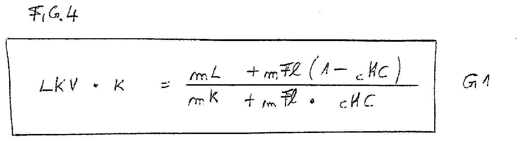

In einem sechsten Schritt V6 wird ein geschätzter Wert W2 der Kohlenwasserstoffkonzentration cHC ermittelt abhängig von dem Massenstrom mFl des Fluids Fl und dem Luft-/Kraftstoff-Verhältnis LKV. Eine Zuordnungsvorschrift zum Ermitteln der Kohlenwasserstoffkonzentration cHC ist in einer Gleichung G1 in der Figur 4 abgebildet. Auf der rechten Seite der Gleichung G1 stehen das Luft-/Kraftstoff-Verhältnis LKV und eine Konstante k, bei der es sich um einen Stöchiometriefaktor handeln kann. Auf der linken Seite der Gleichung G1 stehen zusätzlich zu der Kohlenwasserstoffkonzentration cHC und dem Massenstrom mFL des Fluids Fl ein Massenstrom eingespritzten Kraftstoffs mK sowie ein Massenstrom zugemessener Luft mL. Der Massenstrom zugemessener Luft mL wird über den Lufteinlass 22 in den Ansaugtrakt 14 zugemessen und kann beispielsweise mittels eines Luftmassensensors 72 ermittelt werden.

In einem siebten Schritt V7 wird ein Kalibrieren des Sensors 40 durchgeführt. In diesem Zusammenhang wird eine Zuordnung des Messsignals des Sensors 40 zu erfassten Werten der Kohlenwasserstoffkonzentration cHC angepasst. Dies kann beispielsweise Erfolgen durch Ermitteln eines Korrekturwertes, der dann entsprechend zur Anpassung der erfassten Werte im späteren Betrieb eingesetzt wird. Ferner kann beispielsweise auch ein Kennfeld, das zum Zuordnen des Messsignals des Sensors 40 zu erfassten Werten der Kohlenwasserstoffkonzentration cHC vorgesehen ist, angepasst werden.

Zu diesem Zweck wird ein Zusammenhang zwischen dem erfassten Wert W1 und dem geschätzten Wert W2 ermittelt. Hierfür kann beispielsweise ein Faktor ermittelt werden, mit dem der erfasste Wert W1 auf den geschätzten Wert W2 abgebildet werden kann. Es kann jedoch beispielsweise auch eine Differenz zwischen dem erfassten Wert W1 und dem geschätzten Wert W2 oder eine beliebige andere Zuordnungsvorschrift zwischen dem erfassten Wert W1 und dem geschätzten Wert W2 ermittelt werden. Das Programm kann nach dem siebten Schritt V7 in einem achten Schritt V8 beendet werden. In einer bevorzugten Ausführungsform jedoch fährt das Programm in einem neunten Schritt V9 fort.

In dem neunten Schritt V9 wird ermittelt, ob das Ventil 42 zumindest seit einer vorgegebenen Zeitdauer tmin geöffnet ist. Ist das nicht der Fall, so fährt das Programm in dem neunten Schritt V9 fort. Wenn das Ventil 42 zumindest seit der vorgegebenen Zeitdauer tmin geöffnet ist, dann fährt das Programm in einem zehnten Schritt V10 fort.

In dem zehnten Schritt V10 wird ein weiterer erfasster Wert W3 für die Kohlenwasserstoffkonzentration cHC erfasst.

In dem elften Schritt V11 wird der Sensor 40 kalibriert abhängig von dem weiteren erfassten Wert W3 und einem vorgegebenen Referenzwert WR. In einer bevorzugten Ausführungsform ist der vorgegebene Referenzwert WR charakteristisch für eine minimale Kohlenwasserstoffkonzentration cHC in der Fluidleitung 38. Wenn die vorgegebene Zeitdauer tmin sehr groß gewählt wird, kann beispielsweise davon ausgegangen werden, dass das durch die Fluidleitung 38 strömende Fluid Fl keinen Kohlenwasserstoff mehr enthält. In diesem Fall kann der vorgegebene Referenzwert WR beispielsweise Null sein. Nach Ausführung des elften Schrittes V11 endet das Programm in dem achten Schritt V8.

Eine zusätzliche Ausführung der Schritte V9 bis V11 hat den Vorteil, dass der Sensor 40 sowohl hinsichtlich seines Nullpunktes als auch hinsichtlich der Steigung des Messsignals kalibriert werden kann. Dies ermöglicht sowohl eine Berücksichtigung einer Nullpunktsdrift als auch einer Veränderung der Steigung des Messsignals des Sensors 40.

Die Schritte V3 bis V5 werden derart zeitnah ausgeführt, dass bei der Abarbeitung der Schritte V3 bis V5 die tatsächliche Kohlenwasserstoffkonzentration cHC weitgehend unverändert ist.

## Patentansprüche

1. Verfahren zum Kalibrieren eines Sensors (40) eines Tankentlüftungssystems, das in einer Brennkraftmaschine mit einem Ansaugtrakt (14) angeordnet ist und einen Kohlenwasserstoffspeicher (34) sowie ein Ventil (42) aufweist, wobei das Ventil (42) und der Sensor (40) in einer Fluidleitung (38) angeordnet sind, die Fluidleitung (38) den Kohlenwasserstoffspeicher (34) mit dem Ansaugtrakt (14) koppelt und der Sensor (40) ausgebildet ist zum Erfassen einer Kohlenwasserstoffkonzentration (cHC) eines Fluids (Fl) in der Fluidleitung (38), bei dem
• bei dem geöffneten Ventil (42):
∘ ein erfasster Wert (W1) der Kohlenwasserstoffkonzentration (cHC) erfasst wird mittels des Sensors (40),
∘ ein Massenstrom (mFl) des durch die Fluidleitung (38) in den Ansaugtrakt (14) strömenden Fluids (Fl) erfasst oder ermittelt wird,
∘ von einem der Brennkraftmaschine zur Verbrennung zugeführten Luft-/Kraftstoff-Gemisch das Luft-/Kraftstoff-Verhältnis (LKV) ermittelt wird,
• abhängig von dem Massenstrom (mFl) des Fluids (Fl), dem Luft-/Kraftstoff-Verhältnis (LKV), einem Massenstrom eingespritzten Kraftstoffs (mK) und einem Massenstrom zugemessener Luft (mL) ein geschätzter Wert (W2) der Kohlenwasserstoffkonzentration (cHC) in der Fluidleitung (38) ermittelt wird und
• der Sensor (40) kalibriert wird abhängig von dem erfassten Wert (W1) der Kohlenwasserstoffkonzentration (cHC) und dem geschätzten Wert (W2) der Kohlenwasserstoffkonzentration (cHC).

2. Verfahren nach Anspruch 1, bei dem das Luft-/Kraftstoff-Verhältnis (LKV) ermittelt wird abhängig von einem Messsignal einer Abgassonde in einem Abgastrakt (16) der Brennkraftmaschine.

3. Verfahren nach einem der vorstehenden Ansprüche, bei dem
• ermittelt wird, ob das Ventil (42) zumindest seit einer vorgegebenen Zeitdauer (tmin) geöffnet ist und,
falls das Ventil (42) zumindest seit der vorgegebenen Zeitdauer (tmin) geöffnet ist:
• ein weiterer erfasster Wert (W3) der Kohlenwasserstoffkonzentration (cHC) erfasst wird mittels des Sensors (40),
• der Sensor (40) kalibriert wird abhängig von dem weiteren erfassten Wert (W3) der Kohlenwasserstoffkonzentration (cHC) und einem vorgegebenen Referenzwert (WR).

4. Vorrichtung zum Kalibrieren eines Sensors (40) eines Tankentlüftungssystems, das in einer Brennkraftmaschine mit einem Ansaugtrakt (14) angeordnet ist und einen Kohlenwasserstoffspeicher (34) sowie ein Ventil (42) aufweist, wobei das Ventil (42) und der Sensor (40) in einer Fluidleitung (38) angeordnet sind, die Fluidleitung (38) den Kohlenwasserstoffspeicher (34) mit dem Ansaugtrakt (14) koppelt und der Sensor (40) ausgebildet ist zum Erfassen einer Kohlenwasserstoffkonzentration (cHC) eines Fluids (Fl) in der Fluidleitung (38), wobei die Vorrichtung dazu ausgebildet ist,
• bei dem geöffneten Ventil (42):
∘ einen erfassten Wert (W1) der Kohlenwasserstoffkonzentration (cHC) mittels des Sensors (40) zu erfassen,
∘ einen Massenstrom (mFl) des durch die Fluidleitung (38) in den Ansaugtrakt (14) strömenden Fluids (Fl) zu erfassen oder zu ermitteln,
∘ von einem der Brennkraftmaschine zur Verbrennung zugeführten Luft-/Kraftstoff-Gemisch das Luft-/ Kraftstoff-Verhältnis (LKV) zu ermitteln,
• abhängig von dem Massenstrom (mFl) des Fluids (Fl), dem Luft-/Kraftstoff-Verhältnis (LKV), einem Massenstrom eingespritzten Kraftstoffs (mK) und einem Massenstrom zugemessener Luft (mL) einen geschätzten Wert (W2) der Kohlenwasserstoffkonzentration (cHC) in der Fluidleitung (38) zu ermitteln und
• den Sensor (40) zu kalibrieren abhängig von dem erfassten Wert (W1) der Kohlenwasserstoffkonzentration (cHC) und dem geschätzten Wert (W2) der Kohlenwasserstoffkonzentration (cHC).

## Claims

1. Method for calibrating a sensor (40) of a tank ventilation system which is arranged in an internal combustion engine with an intake section (14) and has a hydrocarbons storage means (34) and also a valve (42), with the valve (42) and the sensor (40) being arranged in a fluid line (38), the fluid line (38) coupling the hydrocarbons storage means (34) to the intake section (14) and the sensor (40) being designed to detect a concentration of hydrocarbons (cHC) in a fluid (Fl) in the fluid line (38), in which method
• when the valve (42) is open:
∘ a detected value (W1) for the concentration of hydrocarbons (cHC) is detected by means of the sensor (40),
∘ a mass flow (mFl) of the fluid (Fl) flowing through the fluid line (38) into the intake section (14) is detected or determined,
∘ the air/fuel ratio (LKV) is determined from an air/fuel mixture which is supplied to the internal combustion engine for combustion purposes,
• an estimated value (W2) for the concentration of hydrocarbons (cHC) in the fluid line (38) is determined as a function of the mass flow (mFl) of the fluid (F1), the air/fuel ratio (LKV), a mass flow of injected fuel (mK) and a mass flow of metered air (mL), and
• the sensor (40) is calibrated as a function of the detected value (W1) for the concentration of hydrocarbons (cHC) and the estimated value (W2) for the concentration of hydrocarbons (cHC).

2. Method according to Claim 1, in which the air/fuel ratio (LKV) is determined as a function of a measurement signal from an exhaust gas probe in an exhaust gas section (16) of the internal combustion engine.

3. Method according to one of the preceding claims, in which
• a check is made to determine whether the valve (42) has been open at least for a prespecified time period (tmin), and
if the valve (42) has been open at least for the prespecified time period (tmin):
• a further detected value (W3) for the concentration of hydrocarbons (cHC) is detected by means of the sensor (40),
• the sensor (40) is calibrated as a function of the further detected value (W3) for the concentration of hydrocarbons (cHC) and a prespecified reference value (WR).

4. Device for calibrating a sensor (40) of a tank ventilation system which is arranged in an internal combustion engine with an intake section (14) and has a hydrocarbons storage means (34) and also a valve (42), with the valve (42) and the sensor (40) being arranged in a fluid line (38), the fluid line (38) coupling the hydrocarbons storage means (34) to the intake section (14) and the sensor (40) being designed to detect a concentration of hydrocarbons (cHC) in a fluid (Fl) in the fluid line (38), with the device being designed
• when the valve (42) is open:
∘ to detect a detected value (W1) for the concentration of hydrocarbons (cHC) by means of the sensor (40),
∘ to detect or to determine a mass flow (mFl) of the fluid (Fl) flowing through the fluid line (38) into the intake section (14),
∘ to determine the air/fuel ratio (LKV) from an air/fuel mixture which is supplied to the internal combustion engine for combustion purposes,
• to determine an estimated value (W2) for the concentration of hydrocarbons (cHC) in the fluid line (38) as a function of the mass flow (mFl) of the fluid (Fl), the air/fuel ratio (LKV), a mass flow of injected fuel (mK) and a mass flow of metered air (mL), and
• to calibrate the sensor (40) as a function of the detected value (W1) for the concentration of hydrocarbons (cHC) and the estimated value (W2) for the concentration of hydrocarbons (cHC).

## Revendications

1. Procédé d'étalonnage d'un capteur (40) d'un système d'aération de réservoir, lequel est disposé dans un moteur à combustion interne avec une voie d'admission (14) et possède un réservoir à hydrocarbure (34) ainsi qu'une soupape (42), la soupape (42) et le capteur (40) étant disposés dans une conduite à fluide (38), la conduite à fluide (38) reliant le réservoir à hydrocarbure (34) à la voie d'admission (14) et le capteur (40) étant configuré pour détecter une concentration d'hydrocarbure (cHc) d'un fluide (Fl) dans la conduite à fluide (38), procédé selon lequel
* lorsque la soupape (42) est ouverte :
∘ une valeur détectée (W1) de la concentration d'hydrocarbure (cHc) est détectée au moyen du capteur (40),
∘ un débit massique (mF1) du fluide (Fl) qui s'écoule à travers la conduite à fluide (38) dans la voie d'admission (14) est détecté ou déterminé,
∘ le rapport air/carburant (LKV) d'un mélange air/carburant acheminé au moteur à combustion interne en vue de la combustion est déterminé,
* une valeur estimée (W2) de la concentration d'hydrocarbure (cHc) dans la conduite à fluide (38) est déterminée suivant le débit massique (mF1) du fluide (Fl), le rapport air/carburant (LKV), un débit massique de carburant (mK) injecté et un débit massique d'air (mL) dosé et
* le capteur (40) est étalonné en fonction de la valeur détectée (W1) de la concentration d'hydrocarbure (cHc) et de la valeur estimée (W2) de la concentration d'hydrocarbure (cHc).

2. Procédé selon la revendication 1, selon lequel le rapport air/carburant (LKV) est déterminé en fonction d'un signal de mesure d'une sonde de gaz d'échappement dans une voie d'échappement (16) du moteur à combustion interne.

3. Procédé selon l'une des revendications précédentes, selon lequel
* il est déterminé si la soupape (42) est ouverte au moins depuis une durée (tmin) prédéfinie et
si la soupape (42) est ouverte au moins depuis la durée (tmin) prédéfinie :
* une valeur détectée supplémentaire (W3) de la concentration d'hydrocarbure (cHc) est détectée au moyen du capteur (40),
* le capteur (40) est étalonné en fonction de la valeur détectée supplémentaire (W3) de la concentration d'hydrocarbure (cHc) et d'une valeur de référence (WR) prédéfinie.

4. Dispositif d'étalonnage d'un capteur (40) d'un système d'aération de réservoir, lequel est disposé dans un moteur à combustion interne avec une voie d'admission (14) et possède un réservoir à hydrocarbure (34) ainsi qu'une soupape (42), la soupape (42) et le capteur (40) étant disposés dans une conduite à fluide (38), la conduite à fluide (38) reliant le réservoir à hydrocarbure (34) à la voie d'admission (14) et le capteur (40) étant configuré pour détecter une concentration d'hydrocarbure (cHc) d'un fluide (Fl) dans la conduite à fluide (38), le dispositif étant configuré pour,
* lorsque la soupape (42) est ouverte :
∘ détecter une valeur détectée (W1) de la concentration d'hydrocarbure (cHc) au moyen du capteur (40),
∘ détecter ou déterminer un débit massique (mF1) du fluide (Fl) qui s'écoule à travers la conduite à fluide (38) dans la voie d'admission (14),
∘ déterminer le rapport air/carburant (LKV) d'un mélange air/carburant acheminé au moteur à combustion interne en vue de la combustion,
* suivant le débit massique (mF1) du fluide (Fl), le rapport air/carburant (LKV), un débit massique de carburant (mK) injecté et un débit massique d'air (mL) dosé, déterminer une valeur estimée (W2) de la concentration d'hydrocarbure (cHc) dans la conduite à fluide (38) et
* étalonner le capteur (40) en fonction de la valeur détectée (W1) de la concentration d'hydrocarbure (cHc) et de la valeur estimée (W2) de la concentration d'hydrocarbure (cHc).
